# EUROPÄISCHE PATENTANMELDUNG

(11) **EP 2 916 249 A1**
(43) Veröffentlichungstag der Anmeldung: **09.09.2015**
(21) Anmeldenummer: 14158098.5
(22) Anmeldetag: 06.03.2014
(51) Int. Cl.: G06F 19/00

(54) **Servicesystem**

(71) Anmelder: Medela Holding AG, 6340 Baar (CH)
(72) Erfinder: Haas, Matthias, 8942 Oberrieden (CH); Weber, Beda, 5643 Sins (CH)
(74) Vertreter: Clerc, Natalia

(57) **Zusammenfassung**

Ein Servicesystem im Umfeld der menschlichen Muttermilchgewinnung oder im Umfeld der medizinischen Drainage weist eine einem Individuum oder einer Entität zugeordnete Basiseinheit (2) und mindestens einen mobilen Datenträger (10) auf. Der mobile Datenträger (10) ist von einem Nutzer einerseits mit Produkten (80, 82, 83) im genannten Umfeld und andererseits mit der Basiseinheit (2) in Daten-kommunizierende Verbindung bringbar. Die Basiseinheit (2) ist vom Nutzer mit einer externen Informations- und Datenplattform (3) in kommunizierende Verbindung bringbar. Das erfindungsgemässe System bietet im genannten Umfeld eine optimale Betreuung und Unterstützung von Nutzern.

## Beschreibung

### TECHNISCHES GEBIET

Die vorliegende Erfindung betrifft ein System im Umfeld der menschlichen Muttermilchgewinnung oder im Umfeld der medizinischen Drainage sowie eine Basiseinheit und einen mobilen Datenträger eines derartigen Systems und ein Produkt, insbesondere eine Saugpumpe, zur Verwendung in einem derartigen System. Das System eignet sich insbesondere für den Bereich von Brustpumpen zum Abpumpen von menschlicher Muttermilch sowie für Thoraxdrainagepumpen und/oder Wunddrainagepumpen.

### STAND DER TECHNIK

Im Umfeld der Muttermilchgewinnung wie auch im Umfeld der medizinischen Drainage wurden in den letzten Jahren neue Erkenntnisse erhalten und eine Vielzahl Produkte in diesem Umfeld wurden weiterentwickelt bzw. neu geschaffen. Insbesondere Saugpumpen wie Brustpumpen, Thoraxdrainagepumpen und Wunddrainagepumpen haben sich in den letzten Jahren massiv weiterentwickelt.

So ist aus WO 01/47577 eine Brustpumpe bekannt, welche unterschiedliche Saugkurven aufweist, die sich nicht nur in Frequenz und maximalem Unterdruck voneinander unterscheiden. Zudem kann die Benützerin der Steuerung der Pumpe neue Saugkurven zuführen. Diese Brustpumpe ist auf dem Markt unter dem Namen "Symphony" bekannt.

Des Weiteren wird versucht, neue Erkenntnisse über die Bereitstellung von Muttermilch in der Brust sowie das Trinkverhalten der Säuglinge zur erhalten, um diese Erkenntnisse in die Weiterentwicklung von Brustpumpen, Brusthauben, Saugern und Säuglings-Trinkflaschen einzubringen. Diese Erkenntnisse basieren im Wesentlichen aus einer Mischung von Erfahrungswerten und wissenschaftlicher Forschung. Das Optimum ist jedoch jeweils von Mutter und Säugling abhängig und deshalb individuell verschieden. Gleiches gilt auch für medizinische Drainagen, insbesondere für die Thoraxdrainage und die Wunddrainage.

Zwar werden üblicherweise Geräte wie Brust- und Drainagepumpen mit Bedienungsanleitungen und Empfehlungen für ihre Benützung versehen. Der zunehmende Einbezug von mehreren Betriebsparametern und weiteren Varianten macht eine optimale Einstellung der Geräte durch den Endbenutzer, beispielsweise die Mutter, oft unmöglich oder unzumutbar. Oft fehlt zudem ein Gesamtüberblick auf der Herstellerseite über die individuellen Bedürfnisse und die Fähigkeit zur individuellen Optimierung auf der Benützerseite.

Im Stand der Technik sind ferner diverse Systeme bekannt, welche sich mit individueller Datenerfassung rund um Schwangerschaft, Geburt und den ersten Lebensmonaten des Babys beschäftigen.

So offenbart WO 2009/143093 ein System zur Erfassung von Laktationsdaten einer Mutter, wobei diese Daten zusammen mit anderen medizinischen Informationen zur Mutter in einer Datenbank gespeichert werden.

In WO 03/034254 werden medizinische Daten vor, während und nach der Schwangerschaft gesammelt und in einer Uhr zur Verfügung gestellt. Auch WO 00/45352 bietet einen Datenspeicher an, in welchen medizinische Daten ab Geburt gespeichert werden. In GB 2 375 181 werden Daten zur Entwicklung des Babys gesammelt und eine automatische Beurteilung des Zustands des Babys gegeben.

DE 101 29 621 und US 2010/0074058 offenbaren Uhren als Orientierungshilfe beim Füttern von Babys. Auch US 2010/0284251 betrifft ein Managementsystem für die Nahrungszufuhr von Babys. In US 2003/0063135 und US 5 691 932 werden auch andere Aspekte der Babybetreuung im Managementsystem mitberücksichtigt.

Diese Vorrichtungen mögen zwar der Mutter und Beratern der Mutter helfen, die Entwicklung des Kindes zu beobachten und einen möglichst geregelten Tagesablauf zu finden. Sie bieten der Mutter jedoch keine Hilfe bei der Auswahl und der Bedienung der zur Verfügung stehenden Produkte im Bereich des Stillens bzw. der Muttermilchgewinnung.

Auch im Bereich der medizinischen Drainage, insbesondere von Thoraxdrainage und Wunddrainage, fehlt eine entsprechende Hilfestellung für das medizinische Personal und den Patienten.

### DARSTELLUNG DER ERFINDUNG

Es ist deshalb eine Aufgabe der Erfindung, ein System im Umfeld der Muttermilchgewinnung oder im Umfeld der medizinischen Drainage zu schaffen, welches eine optimale Nutzung von zur Verfügung stehenden Produkten ermöglicht.

Diese Aufgabe lösen ein System mit den Merkmalen des Patentanspruchs 1, eine Basiseinheit mit den Merkmalen des Patentanspruchs 14, ein mobiler Datenträger mit den Merkmalen des Patentanspruchs 15 sowie ein Produkt zur Verwendung in einem derartigen System mit den Merkmalen des Patentanspruchs 18.

Das erfindungsgemässe Servicesystem weist eine einem Individuum oder einer Entität zugeordnete Basiseinheit und mindestens einen mobilen Datenträger auf. Der mobile Datenträger ist von einem Nutzer einerseits mindestens mit Produkten im Umfeld der Muttermilchgewinnung oder im Umfeld von medizinischen Saugpumpen und andererseits mit der Basiseinheit in Daten-kommunizierende Verbindung bringbar und die Basiseinheit ist vom Nutzer mit einer externen Informations- und Datenplattform in kommunizierende Verbindung bringbar.

Produkte im Sinne dieses Textes sind in einer ersten Ausführungsform käuflich oder zur Miete erhältliche Geräte, Vorrichtungen und Gegenstände eines oder mehrerer Hersteller. In einer anderen Ausführungsform sind Produkte alternativ oder zusätzlich auch gewerblich oder kostenlos angebotene Dienstleistungen, wie beispielsweise Stillberatungen oder Säuglingsernährungsberatungen. Weitere Ausführungsformen beinhalten zusätzlich noch weitere Arten von Produkten.

Das Individuum ist im Falle, dass das System im Stillbereich eingesetzt wird und die Saugpumpe eine Brustpumpe ist, eine zukünftige oder bereits stillende bzw. brustpumpende Mutter oder das Baby. Die Entität kann in diesem Fall beispielsweise ein Spital, ein Geburtshaus, eine Säuglingsabteilung oder eine Neonatologie-Abteilung eines Spitals, eine Stillberaterin mit mehreren zu beratenden Müttern oder eine andere im Beratungsbereich tätige Institution sein. Der Nutzer ist ein Endnutzer, beispielsweise die Mutter, Pflegepersonal, Ärzte, Stillberaterinnen oder andere, die Mutter bei der Betreuung des Babys unterstützende Personen.

Im Falle, dass das System im Bereich der medizinischen Drainage, insbesondere der Thoraxdrainage oder Wunddrainage, eingesetzt wird, ist das Individuum ein Patient. Die Entität ist wiederum ein Spital oder eine Pflegeinstitution bzw. eine Abteilung des Spitals oder der Institution. Der Nutzer ist vorzugsweise ein Arzt oder das Pflegepersonal. Er kann jedoch beispielsweise auch der Patient oder eine ihn pflegende Person sein.

Der mobile Datenträger ist in einer bevorzugten Ausführungsform vom Nutzer mit einem oder mehreren der folgenden Produkte in daten-kommunizierende Verbindung bringbar: Brustpumpen, Brusthauben, Sauger, Milchsammelbehälter, Milchaufbewahrungsbehälter, Milchverabreichungsbehälter, Vakuumschläuche, Steckverbindungen. In einer anderen Ausführungsform ist der mobile Datenträger alternativ oder zusätzlich mit einem oder mehreren der folgenden Produkte in daten-kommunizierende Verbindung bringbar: Thoraxdrainagepumpen, Fluidsammelbehälter, Drainage- und Serviceschläuchen, Steckverbindungen. In einer weiteren Ausführungsform ist der mobile Datenträger alternativ oder zusätzlich mit einem oder mehreren der folgenden Produkte in daten-kommunizierende Verbindung bringbar: Wunddrainagepumpen, Wundabdeckungen, Drainageschläuche, Serviceschläuchen, Steckverbindungen.

Vorzugsweise ist die Mobilität des mobilen Datenträgers von der Basiseinheit und/oder von den Produkten unabhängig, so dass der Nutzer, insbesondere das Individuum, den Datenträger stets bei sich tragen kann.

Der mobile Datenträger kann beim Individuum verbleiben, so dass er alternative Geräte und Vorrichtungen, insbesondere neue Saugpumpen ausprobieren kann und diese von Anfang an basierend auf Daten aus früheren Erfahrungen mit Saugpumpen oder Geräten betrieben werden können. Dadurch ist eine schnellere und einfachere Optimierung der Betriebsparameter der neuen Saugpumpe bzw. der neuen Vorrichtung möglich. Beispielsweise kann eine Mutter beim Verlassen des Spitals einen mobilen Datenträger erhalten, auf welchem alle relevanten Daten zu der im Spital verwendeten Brustpumpe gespeichert sind, beispielsweise der Brustpumpentyp und die eingestellten Saugparameter inkl. der allenfalls gewählten Saugkurve. Will sie nun eine eigene Brustpumpe mieten oder kaufen, vereinfacht dies für sie die Auswahl und Inbetriebnahme der Pumpe, selbst wenn sie nicht das identische Modell wie im Spital benützt, kauft bzw. mietet.

Wenn die Mutter das Spital verlässt, können beispielsweise auch individuelle Milchmenge und Abpumpintervalle und Dauer des Abpumpens auf dem mobilen Datenträger gespeichert sein. Auch dies hilft bei der Wahl der Pumpe. Dies sind jedoch auch nützliche Daten für die Stillberaterinnen, welche die weitere Betreuung der Mutter übernehmen.

All diese Daten sind auch während des Spitalaufenthaltes für das Fachpersonal bzw. bei Verwendung einer anderen Saugpumpe im Spital hilfreich. Insbesondere bei Frühgeborenen oder bei kranken bzw. behinderten Kindern können diese Daten auch Informationen zur Aufbereitung der abgepumpten Muttermilch beinhalten.

Der Mutter bzw. dem Baby können mehrere mobile Datenträger zugeordnet sein. Insbesondere können erste Datenträger die Brustpumpe betreffen und zweite Datenträger andere Produkte. Die anderen Produkte können beispielweise ein Zubehörteil, wie beispielsweise ein Milchsammelbehälter, eine Brusthaube, ein Sauger oder eine Milchverabreichungsflasche, d.h. eine Säuglingsflasche, sein. Die Daten zu all diesen Geräten und Vorrichtungen können jedoch auch auf demselben Datenträger gespeichert sein.

Diese auf dem mindestens einen mobilen Datenträger gespeicherten Daten werden der Basiseinheit übermittelt bzw. von der Basiseinheit gelangen weitere Daten auf den mobilen Datenträger. So lassen sich im Falle von mehreren, demselben Individuum zugeordneten Datenträgern Daten und Informationen austauschen, optimieren und auch transformieren. Wird z.B. eine grössere Brusthaube verwendet, so können automatisch Abpumpparameter in der Saugpumpe verändert werden. Ist die Basiseinheit einer Entität mit mehreren Individuen zugeordnet, so lassen sich Erfahrungswerte der einzelnen Individuen auch anderen Individuen zugänglich machen.

Die erfindungsgemässe Basiseinheit ist mit einer externen Informations- und Datenplattform verbunden. Die Plattform kann lokal in einem Spital, einer Pflegeinstitution oder einer anderen ähnlichen Einrichtung vorhanden sein. Vorzugsweise ist sie jedoch internetbasiert, wobei mindestens einzelne Teile davon vorzugsweise in einer Cloud platziert sind. Die Plattform kann jedoch auch ausschliesslich bei einem Produktehersteller lokalisiert sein. Über diese Plattform lassen sich Daten zwischen Individuen bzw. Entitäten und Beratern bzw. Herstellern austauschen, diese Daten lassen sich Individuum-bezogen oder Individuen-übergreifend optimieren und transformieren und auch wieder einzelnen Basiseinheiten übermitteln bzw. zuordnen.

So kann beispielsweise eine Mutter über diese Plattform Hilfe bei einer Stillberaterin suchen, bei einer Fehlfunktion der Pumpe Hilfe beim Hersteller erhalten oder sie kann bei Bestellung von Gebrauchsmaterial von Fehlkäufen geschützt werden. Des Weiteren können Berater und Hersteller anhand des Datenpools Erkenntnisse aus der Praxis erhalten und Rückschlüsse für Beratung und Neuentwicklung von Produkten beziehen. Das System bietet somit im Falle eines suboptimalen Ergebnisses bei Benützung der Produkte eine möglichst schnelle Erkennung und gegebenenfalls Lösung des Problems.

Ein weiterer Vorteil ist, dass die Kundenbindung in allen Bereichen, insbesondere aber betreffend Produkten im Stillbereich, erhöht wird. Die Mutter kann bereits vor der Geburt einen Datenträger erhalten, so dass sie sich frühzeitig mit der Daten- und Informationsplattform bekannt und vertraut machen kann und sich und/oder die Produkte auch in entsprechenden Fachgeschäften mit dem Datenträger identifizieren kann.

In einer bevorzugten Ausführungsform ermöglicht deshalb der mobile Datenträger einen bidirektionalen Datentransfer mit der Basiseinheit, um mindestens einen Teil der oben genannten Funktionen zu ermöglichen.

Vorzugsweise erfolgt die Datenkommunikation zwischen mobilen Datenträgern und Basiseinheit und/oder zwischen mobilen Datenträgern und den Produkten, insbesondere den Saugpumpen, drahtlos. Dies erleichtert die Handhabung.

In einer Ausführungsform ist die Basiseinheit eine eigenständige Einheit mit Internetanschlussfunktion. Sie kann jedoch auch Teil eines Multifunktionskommunikationsmittels, beispielsweise eines Smartphones, sein. Beispielsweise lässt sich ein App auf das Smartphone herunterladen, damit die Kommunikation mit dem mobilen Datenträger und/oder der externen Plattform bereits vor dem Kauf oder der Miete irgendwelcher Vorrichtungen und Geräte erfolgen kann.

Vorzugsweise ermöglicht die Basiseinheit einen bidirektionalen Informations- und Datentransfer mit mindestens einem Bereich der Informations- und Datenplattform, damit mindestens ein Teil der oben beschriebenen Funktionen ausgeübt werden kann.

Vorzugsweise ist die Basiseinheit von der Mutter oder vom Nutzer mit den Produkten, insbesondere den Saugpumpen, in Daten-kommunizierende Verbindung bringbar. Ist die Verbindung bidirektional, lassen sich nicht nur Informationen und Daten von den Produkten herunterladen, insbesondere von den Saugpumpen. Es lassen sich auch neue Informationen, Daten, Betriebsparameter und Betriebsprogramme in die Produkte laden, insbesondere in die Saugpumpen. Insbesondere lassen sich über die Betriebseinheit von der Plattform neuentwickelte Saugkurven oder andere vom Nutzer nachträglich erworbene und/oder von der Plattform herunter geladene neue Daten, Informationen, Parameter oder Programme benützen.

Vorzugsweise weist die Basiseinheit eine Auflagefläche zur Auflage des mobilen Datenträgers zum Zwecke des Datentransfers auf. Es lassen sich auf diese Art beispielsweise auch Energiespeicher der Datenträger, der Produkte, insbesondere der Saugpumpen, und eines Smartphones und anderem auch kabellos aufladen.

Der mobile Datenträger weist vorzugsweise die äussere Form einer Spielmarke (Chip), einer Münze oder einer Kreditkarte auf. In diesen Ausführungsformen ist er relativ kostengünstig und klein, und er lässt sich überall, insbesondere in einer Hand- oder Hosentasche verstauen. Er kann jedoch auch im oder am Produkt selber entfernbar angeordnet oder fest integriert sein. Des Weiteren kann ein Smartphone oder ein anderes Multifunktionskommunikationsmittel den mobilen individuellen Datenträger bilden.

In einer Ausführungsform weist der mobile Datenträger mindestens einen Sensor zur Detektion von Messwerten auf. Dadurch lässt er sich insbesondere bei Verwendung mit einem Milchsammelbehälter oder einem Milchverabreichungsbehälter zur Detektion und/oder Messung der in den Behälter eintretenden bzw. ausfliessenden Milchmenge und/oder der Temperatur nutzen.

Vorzugsweise ist der mobile Datenträger zur Befestigung an einem Produkt zwecks daten-kommunizierender oder messwertdetektierender Verbindung ausgebildet. Entsprechend ist das Produkt, insbesondere die Saugpumpe, zur Aufnahme des mobilen Datenträgers zwecks daten-kommunizierender oder messwertdetektierender Verbindung ausgebildet.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung des erfindungsgemässen Systems im Umfeld der menschlichen Muttermilchgewinnung;
- Figur 2: eine schematische Darstellung einer erfindungsgemässen Basiseinheit mit darauf liegendem mobilen Datenträger;
- Figur 3: eine schematische Darstellung einer erfindungsgemässen Basiseinheit mit darauf liegender Brustpumpe;
- Figur 4: eine schematische Darstellung einer erfindungsgemässen Basiseinheit mit darauf liegendem Smartphone;
- Figur 5: eine erste Kombination des erfindungsgemässen Datenträgers mit einer Brustpumpe;
- Figur 6: eine zweite Kombination des erfindungsgemässen Datenträgers mit einem Milchsammelbehälter;
- Figur 7: eine dritte Kombination des erfindungsgemässen Datenträgers mit einer Brusthaube;
- Figur 8: eine vierte Kombination des erfindungsgemässen Datenträgers mit einer Säuglingsflasche;
- Figur 9: eine schematische Darstellung von zwei Basiseinheiten;
- Figur 10: eine erste erfindungsgemässe Anwendungsmöglichkeit einer Saugpumpe mit einer Basiseinheit;
- Figur 11a: eine zweite erfindungsgemässe Anwendungsmöglichkeit einer Saugpumpe mit einem Datenträger;
- Figur 11b: eine Variante der Anwendungsmöglichkeit gemäss Figur 11a in der Kombination einer Saugpumpe mit einer Basiseinheit;
- Figur 11c: eine Variante der Anwendungsmöglichkeit gemäss Figur 11a in der Kombination einer Saugpumpe mit einer Basiseinheit;
- Figur 11d: eine Variante der Anwendungsmöglichkeit gemäss Figur 11a in der Kombination einer Saugpumpe mit einer Basiseinheit;
- Figur 12a: eine dritte erfindungsgemässe Anwendungsmöglichkeit einer Saugpumpe mit einer Basiseinheit;
- Figur 12b: eine Variante der Anwendungsmöglichkeit gemäss Figur 12a in der Kombination einer Saugpumpe mit einer Basiseinheit;
- Figur 12c: eine Variante der Anwendungsmöglichkeit gemäss Figur 12a in der Kombination einer Saugpumpe mit einer Basiseinheit und einem Datenträger;
- Figur 13: eine vierte erfindungsgemässe Anwendungsmöglichkeit einer Saugpumpe mit einer Basiseinheit;
- Figur 14: eine fünfte erfindungsgemässe Anwendungsmöglichkeit einer Saugpumpe mit einem Datenträger;
- Figur 15a: eine sechste erfindungsgemässe Anwendungsmöglichkeit einer Saugpumpe mit einem Datenträger;
- Figur 15b: eine Variante der Anwendungsmöglichkeit gemäss Figur 15a;
- Figur 16: eine siebte erfindungsgemässe Anwendungsmöglichkeit einer Saugpumpe mit einer Basiseinheit und einem Datenträger;
- Figur 17a: eine achte erfindungsgemässe Anwendungsmöglichkeit eines Datenträgers mit einer Basiseinheit;
- Figur 17b: eine Variante der Anwendungsmöglichkeit gemäss Figur 17a;
- Figur 18a: eine neunte erfindungsgemässe Anwendungsmöglichkeit einer Saugpumpe mit einer Basiseinheit;
- Figur 18b: eine Variante der Anwendungsmöglichkeit gemäss Figur 18a;
- Figur 19a: eine zehnte erfindungsgemässe Anwendungsmöglichkeit einer Saugpumpe mit einer Basiseinheit;
- Figur 19b: eine Variante der Anwendungsmöglichkeit gemäss Figur 19a und
- Figur 20: eine elfte erfindungsgemässe Anwendungsmöglichkeit einer Saugpumpe mit einer Basiseinheit und einem Datenträger.

### BESCHREIBUNG BEVORZUGTER AUSFÜHRUNGSFORMEN

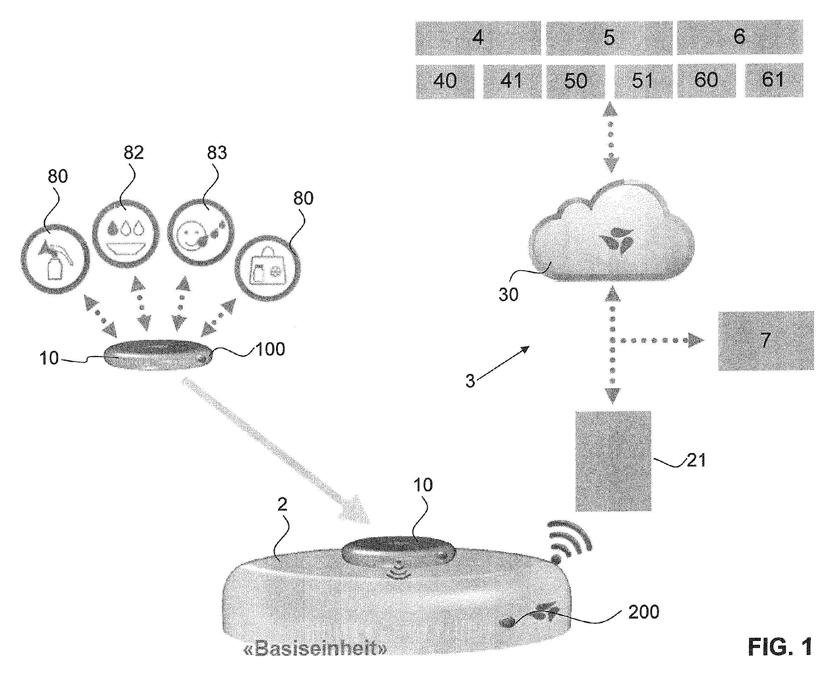

In Figur 1 ist schematisch ein erfindungsgemässes Service-System im Umfeld der Muttermilchgewinnung dargestellt. Das hier Gesagte lässt sich jedoch analog auf das Umfeld der medizinischen Drainage, insbesondere der Thoraxdrainage und der Wunddrainage, übertragen.

Das System weist mindestens einen mobilen Datenträger 10, im Folgenden auch Chip genannt, und mindestens eine Basiseinheit 2 auf. Vorzugsweise umfasst das System mehrere Basiseinheiten 2 und mehrere Chips 10.

Der Datenträger 10 ist in diesem Beispiel in Form und Grösse einer flachen runden Spielmarke ausgebildet. Er kann jedoch auch andere Grössen und Formen aufweisen. Vorzugsweise ist er leicht, robust und klein ausgestaltet, so dass er auch in einer Hosentasche oder einem Geldbeutel Platz findet. Er kann auch als Anhänger ausgebildet sein, sei es beispielsweise für einen Schlüsselring oder für eine Halskette.

Der mobile Datenträger 10 verfügt über einen Datenspeicher und die Möglichkeit zur aktiven und/oder passiven Datenübertragung. Ein Mikroprozessor mit wenigen Kombinations- und Auswertungsmöglichkeiten ist vorzugsweise ebenfalls vorhanden. Der mobile Datenträger 10 verfügt somit über keine oder nur eine geringe Intelligenz.

Wie in Figur 1 schematisch dargestellt, ist der Datenträger 10 mit diversen Produkten in daten-kommunizierende Verbindung bringbar. Diese Produkte sind beispielsweise eine manuelle oder motorisch betriebene Brustpumpe 80, ein Milchsammelbehälter 82 oder eine Milchverabreichungseinheit 83. Es ist auch möglich, dass ein- und derselbe Datenträger 10 mit allen diesen Produkten in kommunizierende Verbindung bringbar ist, jedoch vorzugsweise nacheinander. Die uni- oder bidirektionale (d.h. die in einer oder zwei Richtungen erfolgende) Kommunikation mit den Produkten erfolgt vorzugsweise drahtlos mit üblichen bekannten Mitteln.

Der Datenträger 10 verfügt vorzugsweise über mindestens eine Anzeige, hier eine optische Anzeige, zum Beispiel eine Leuchtdiode oder eine LCD 100, welche beispielsweise durch Blinken oder konstantes Leuchten anzeigt, dass ein Datentransfer stattfindet bzw. dass er abgeschlossen ist.

Je nach Anwendungsbereich des Datenträgers 10 kann er zudem mit einem Energiespeicher ausgestattet sein. Der Energiespeicher ist vorzugsweise wieder aufladbar.

Der mobile Datenträger 10 ist einem Individuum zugeordnet. Das Individuum ist im Umfeld der Muttermilchgewinnung üblicherweise eine Mutter oder ein Säugling. Die Zuordnung kann rein dadurch erfolgen, dass der Chip 10 im Besitz des Individuums ist. Es können, aber es müssen nicht, Individuum-spezifische Identifikationsdaten auf dem Chip 10 gespeichert sein. Der Chip 10 lässt sich beispielsweise auch lediglich über einen Chipspezifischen Identifikationscode zuordnen. Der Chip 10 kann zudem einer Mutter oder einem Säugling zugeordnet sein, jedoch im Besitz eines die Mutter in der Betreuung unterstützenden Helfers, z.B. des Vaters oder einer Krankenschwester, sein. Die Nutzer sind somit nicht nur Mütter, sondern auch andere Personen.

Die Nutzer erhalten die Chips 10 auf verschiedene Weise. So kann einer Mutter bereits in der Schwangerschaftsbetreuung ein Chip oder mehrere derartige Chips ausgehändigt werden. Sie kann ihn während des Spitalaufenthaltes bei der Geburt oder bei Verlassen des Spitals erhalten. Sie kann ihn auch von einem Vertreiber der Umfeld-spezifischen Produkte, von einem Arzt oder einer Still- bzw. Säuglingsernährungsberaterin erhalten.

Im Drainagebereich ist das Individuum analog ein Patient, die Nutzer sind Patienten, Ärzte, Pflegepersonal, Angehörige und Bekannte. Üblicherweise, aber nicht ausschliessend, wird der Chip vom Spital, der Pflegeinstitution oder vom Arzt erhalten.

Die Nutzung des Chips 10 wird später im Text beschrieben.

Das erfindungsgemässe System umfasst auch mindestens eine Basiseinheit 2. Diese Basiseinheit 2 ist einem Individuum oder einer Entität zugeordnet. Das Individuum wird bereits oben beschrieben. Die Entität ist üblicherweise ein Spital oder eine Pflege- oder Beratungsinstitution, insbesondere eine Stillberaterin. Die Entität betreut mehrere Individuen, welche über die oben genannten mobilen Datenträger 10 verfügen.

Die Basiseinheit 2 ist üblicherweise stationär, wobei sie üblicherweise klein genug ist, um von einer Lokalität zu einer anderen transportiert zu werden. Die Basiseinheit 2 verfügt vorzugsweise über einen Internetanschluss, um mit einer externen Informations- und Datenplattform 3 zu kommunizieren und Daten sowie Informationen auszutauschen. Die Basiseinheit 2 kann üblicherweise mit einer Mehrzahl von Chips 10 uni- oder bidirektional kommunizieren, d.h. Daten von den Chips abrufen und/oder empfangen. Sie ist insbesondere in der Lage, die Chips 10 zu identifizieren und sie dem Individuum zuzuordnen.

Die Basiseinheit 2 verfügt vorzugsweise über einen Datenspeicher, um Daten von den Chips 10, aber auch von der Informations- und Datenplattform 3 zu speichern. Auch die Basiseinheit 2 weist vorzugsweise mindestens eine Anzeige, hier eine Leuchtdiode oder LCD 200, auf, um die Kommunikation und/oder den Abschluss des Datentransfers anzuzeigen. Auch hier erfolgt die uni- oder bidirektionale Kommunikation mit den Chips 10 vorzugsweise drahtlos über bekannte Mittel.

Die uni- oder bidirektionale Kommunikation mit der Informations- und Datenplattform 3 kann drahtlos oder über eine Kabelverbindung erfolgen. Es lässt sich auch eine Verbindungseinheit 21, beispielsweise ein Smartphone hierzu verwenden. Die Basiseinheit 2 verfügt vorzugsweise über eine grössere Intelligenz als die Chips 10, d.h. sie kann beispielsweise Chips und Daten identifizieren, auswählen, gruppieren, verändern und neu zuordnen.

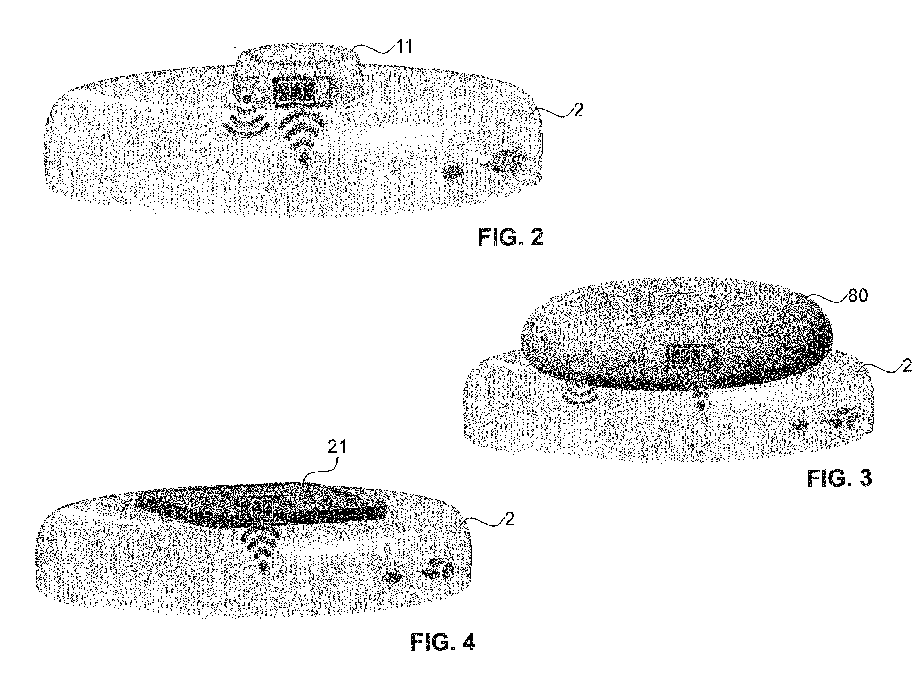

In Figur 2 ist ein mobiler Datenträger in Form eines Datenträgerrings 11 auf eine Oberfläche, vorzugsweise eine Auflagefläche mit integriertem Sensor, der Basiseinheit 2 aufgelegt und übermittelt sowie empfängt Daten.

Die Basiseinheit 2 ist vorzugsweise auch mit den Produkten in uni- oder bidirektionaler datenkommunizierende Verbindung bringbar. Dies ist in Figur 3 dargestellt. Eine Brustpumpe 80, hier eine tragbare kompakte Brustpumpe, liegt auf der oben genannten Kontaktfläche der Basiseinheit 2 und übermittelt sowie empfängt Daten.

In Figur 4 ist dargestellt, dass die Basiseinheit 2 Daten an eine Verbindungseinheit 21, hier ein Smartphone, übermittelt.

In allen drei Fällen gemäss den Figuren 2 bis 4 wird in einer bevorzugten Ausführungsform auch gleich ein Energiespeicher des aufliegenden Elements mittels der Basiseinheit 2 geladen. Dies macht insbesondere dann Sinn, wenn die Basiseinheit 2 selber mit dem Stromversorgungsnetz verbunden ist.

In all diesen Fällen bestimmt der Nutzer durch Auflegen des Elements auf die Basiseinheit, ob und wann die Daten und Informationen übermittelt werden. Diese Übermittlung der Daten und Informationen kann je nach Ausführungsform automatisch und/oder durch spezielle Aktivierung erfolgen.

Wie in Figur 1 dargestellt ist, befindet sich die Informations- und Datenplattform 3 vorzugsweise mindestens teilweise in einer Cloud 30. Clouds sind mittlerweile wohlbekannte IT-Infrastrukturen, welche dynamisch an den Bedarf angepasst über ein Netzwerk zur Verfügung gestellt werden. Auf diese erfindungsgemässe Cloud 30 haben unterschiedliche Kreise Zugriff: einerseits die diversen Basiseinheiten 2, wobei in Figur 1 symbolisch für alle eine einzige Basiseinheit 2 dargestellt ist. Des Weiteren haben Produktanwender bzw. Nutzer 4 Zugriff, wie beispielsweise Mütter 40 und deren Helfer 41, um Daten und Informationen abzufragen und auf die Plattform 3 zu laden. Unterstützungseinheiten 5 wie Spitäler, Pflegeeinheiten, Stillberaterinnen 50 und andere Beratungsdienste 51 können Informationen und Daten von der Plattform 3 laden und in diese eingeben. Hersteller 6, z.B. deren Kundendienste 60 und interne Bereiche 61 wie Marketing, Qualitätssicherung, Kundenservice und Entwicklungsabteilungen, können das System ebenfalls bidirektional nutzen. Die Plattform 3 kann zudem mit einem oder mehreren elektronischen Verkaufsstellen 7 (e-Shops) in Verbindung stehen oder beinhalten. Andere Kreise können ebenfalls einen uni- oder bidirektionalen Zugriff erhalten, beispielsweise Forschungsstätten oder Hochschulen.

In der Plattform 3 werden Daten ausgetauscht, transformiert, optimiert und auch wieder einzelnen Basiseinheiten zugeordnet. Einzelne andere Kreise können ebenfalls derart geänderte Daten und Informationen automatisch oder auf Anfrage beziehen. Des Weiteren dient die Plattform 3 für den direkten und vereinfachten Austausch zwischen einem einzelnen Individuum oder Entität und einem anderen Kreis bzw. zwischen Individuen oder Entitäten untereinander. Auch dies wird nachfolgend noch genauer erläutert.

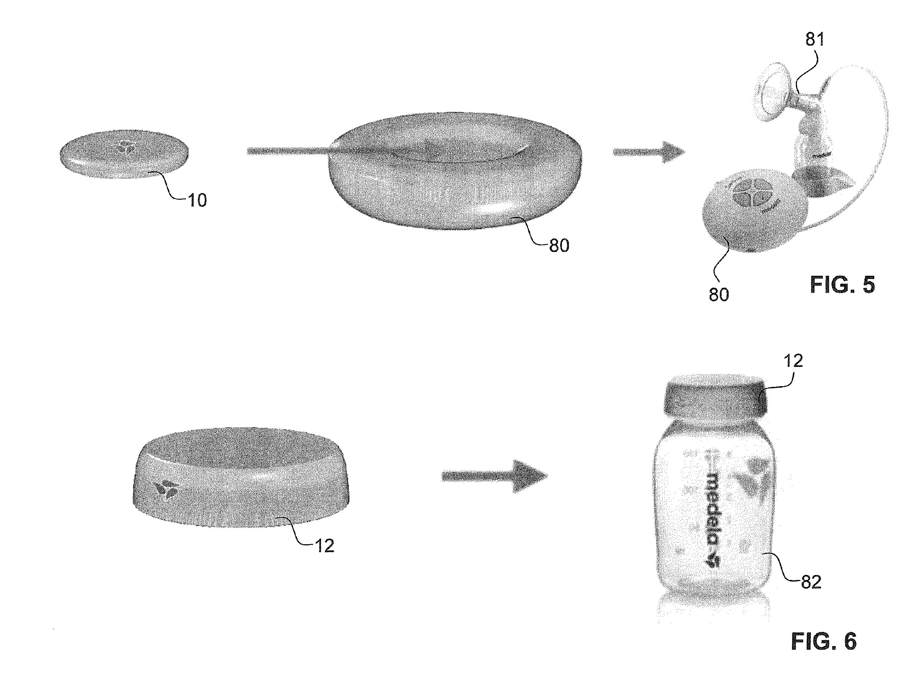

In Figur 5 ist eine mögliche Ausgestaltung von Chip 10 und Brustpumpe 80 dargestellt. Der Chip 10 ist rund und flach wie eine Münze, die Brustpumpe 80 weist eine entsprechende Vertiefung auf. Mögliche Datentransfers werden hier später beschrieben.

In Figur 6 ist der mobile Datenträger 12 in Form eines Deckels für einen Milchsammelbehälter 82, hier in Form einer Flasche, ausgebildet. Der Datenträger 12 beinhaltet vorzugsweise Daten zur Abfüllzeit, evtl. auch der Füllmenge oder der Temperatur. Alternativ oder zusätzlich kann er Daten und Informationen zur Aufbereitung der Milch zwecks ihrer Verabreichung enthalten. Diese Daten können der Basiseinheit 2, aber auch einem Produkt, beispielsweise einer Milchverabreichungsflasche oder einem Milchwärmer, übermittelt werden.

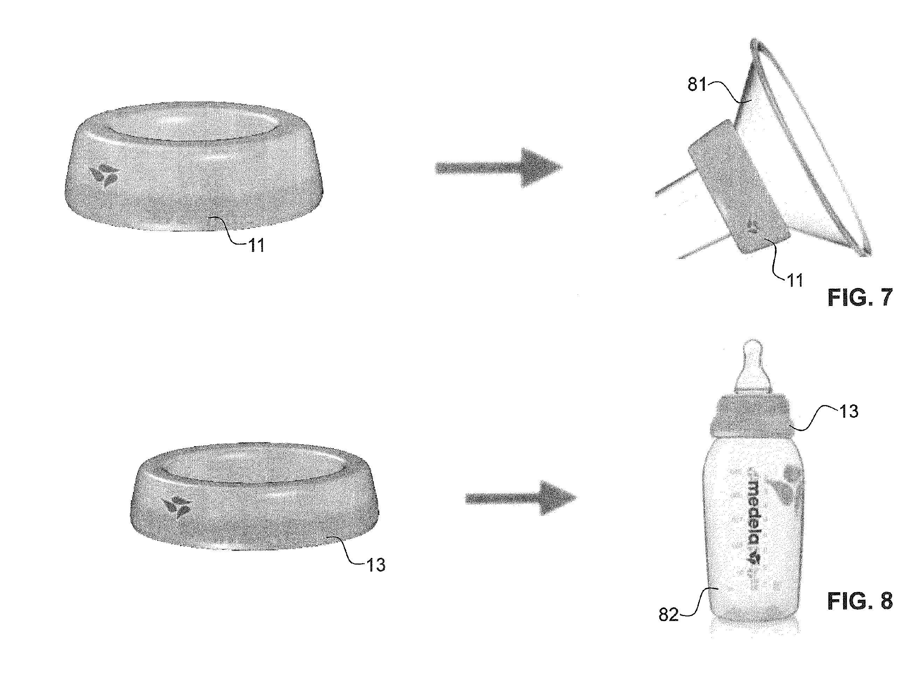

In Figur 7 ist der mobile Datenträger 11 ein Ring, welcher auf eine Brusthaube 81 aufsetzbar ist. Üblicherweise weisen die Brusthauben 81 einen Trichter zur Auflage auf die Mutterbrust und einen nachfolgenden zylinderförmigen Teil auf. Über diesen zylinderförmigen Teil lässt sich der Datenträger 11 stülpen und am Trichter anlegen. Mittels Sensoren im Datenträgerring 11 oder in der Brusthaube selber lässt sich die abgepumpte Milchmenge detektieren und diese Daten lassen sich an die Basiseinheit oder einem der Produkte übermitteln. Ist der Sensor in der Brusthaube, werden die Daten zuerst an den Ring 11 übermittelt. Der Datenträgerring 11 weist hier vorzugsweise einen Energiespeicher auf.

In Figur 8 ist ebenfalls ein Datenträger in Form eines Rings 13 dargestellt. Es kann der identische Ring wie in Figur 7 sein oder ein anders aufgebauter. Hier wird er mit einer Säuglingsflasche 82 verwendet. Er misst den die Flasche verlassenden Milchfluss bzw. die die Flasche verlassende Milchmenge. Der Sensor hierzu kann im Datenträger, in der Flasche oder im Sauger angeordnet sein. Auch hier weist der Ring 13 vorzugsweise einen Energiespeicher auf.

Anhand der nachfolgend beschriebenen Figuren werden einige Anwendungsbeispiele erläutert. Andere Einsatzmöglichkeiten bestehen. Die Anwendungsbeispiele lassen sich vorzugsweise in einer einzigen Ausführungsform des erfindungsgemässen Systems verwirklichen. In anderen Ausführungsformen des Systems ist jedoch nur eine einzige dieser Möglichkeiten oder Unterkombinationen verwirklicht.

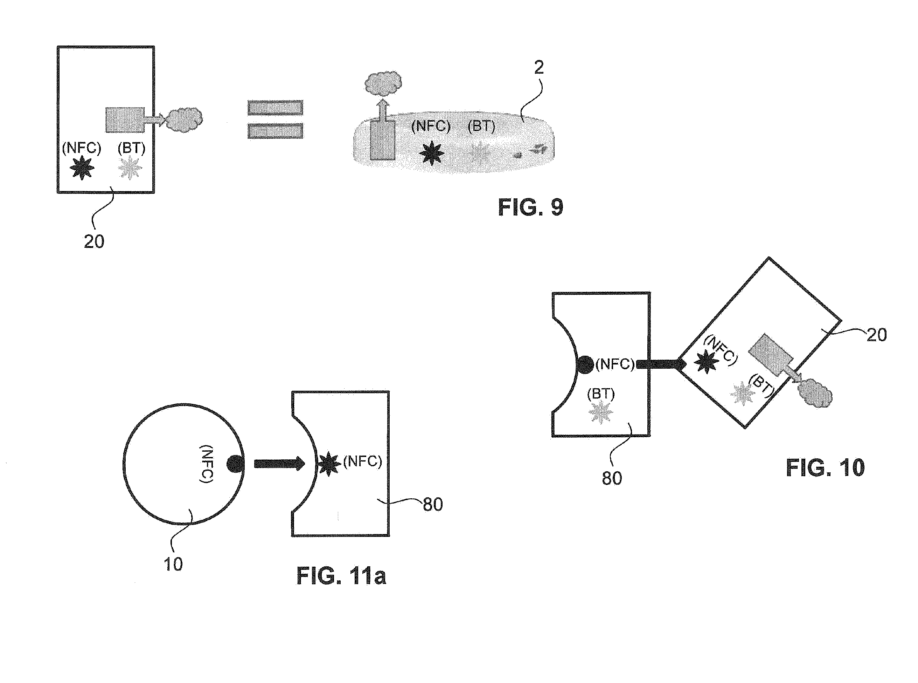

In Figur 9 ist dargestellt, dass die Basiseinheit 2 einerseits ein separates Teil sein kann oder auch in einem Smartphone 20 oder einem anderen Multikommunikationsgerät integriert sein kann, beispielsweise als App. Auch der mobile Datenträger kann entweder in einem Produkt integriert oder in einem Smartphone oder einem anderen Multikommunikationsgerät verwirklicht sein, beispielsweise ebenfalls als App.

In den nachfolgenden Abbildungen ist jeweils ein Smartphone 20 dargestellt, wobei die nachfolgende Offenbarung identisch auf andere Basiseinheiten zu übertragen ist. Zudem ist als Produkt vor allem eine Brustpumpe erläutert. Die Kommunikationsarten und Anwendungen lassen sich auch auf andere Produkte übertragen.

Figur 10 zeigt eine erste Anwendung: die Brustpumpe 80 ist mit einem NFC Tag (Nahfeldkommunikations-Etikett) ausgerüstet. Mittels Smartphone 20 werden Herstellerdaten ausgelesen und Individuum-spezifisch gespeichert. Die Daten können über Internet an die Cloud übermittelt werden und so dem Hersteller oder einem anderen Kreis mitgeteilt werden.

In Figur 11a verfügt der Chip 10 über Individuum-spezifische Daten für ein optimiertes Abpumpen. Die Brustpumpe 80 weist einen aktiven NFC Tag Leser auf, um diese Daten vom Chip 10 zu lesen.

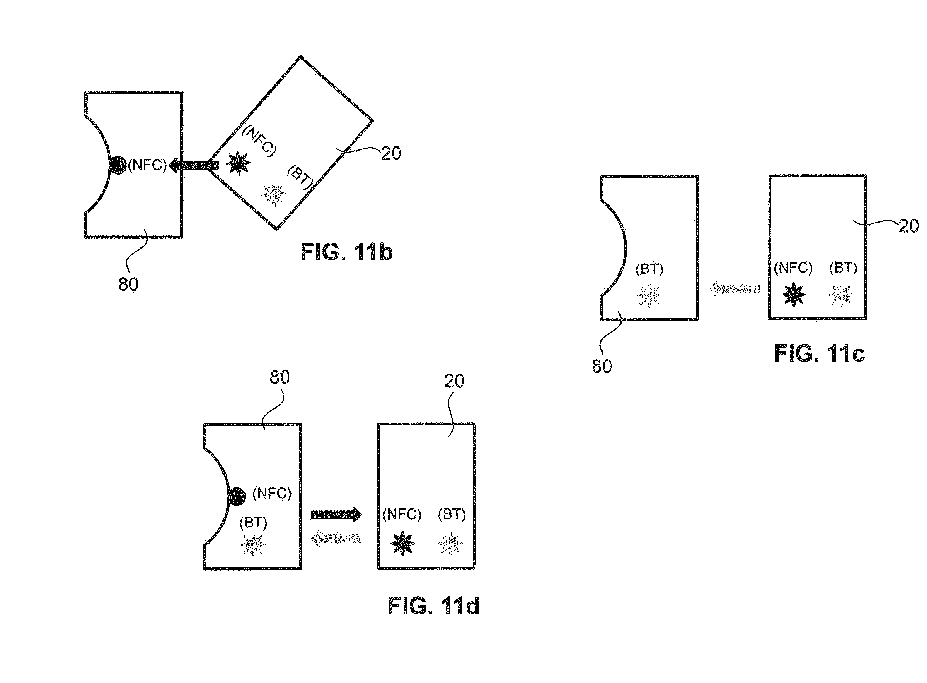

In Figur 11b ist die Brustpumpe 80 mit einem NFC Tag ausgestattet, so dass sie Daten vom Smartphone 20 empfangen kann.

In Figur 11c ist die Pumpe 80 mit einem Bluetooth Modul (BT) ausgerüstet und kommuniziert mit dem Smartphone 20, so dass das Smartphone 20 Daten und Informationen an die Pumpe 80 übermitteln kann.

In Figur 11d weist die Pumpe 80 einen passiven NFC Tag und ein Bluetooth Modul auf. Das NFC Tag konfiguriert ein Smartphone 20 App, welches seinerseits die Pumpe via Bluetooth konfiguriert.

In diesen Beispielen gemäss den Figuren 11a bis 11d wird die Pumpe beim Benützer konfiguriert. Beispielsweise werden für das Individuum optimale Betriebsparameter oder optimierte Programme geladen oder es werden generell neuentwickelte Programme und/oder Betriebsparameter installiert. Der Nutzer kann somit eine bestehende Pumpe mit neuen und/oder verbesserten bzw. optimierten Funktionen aufrüsten.

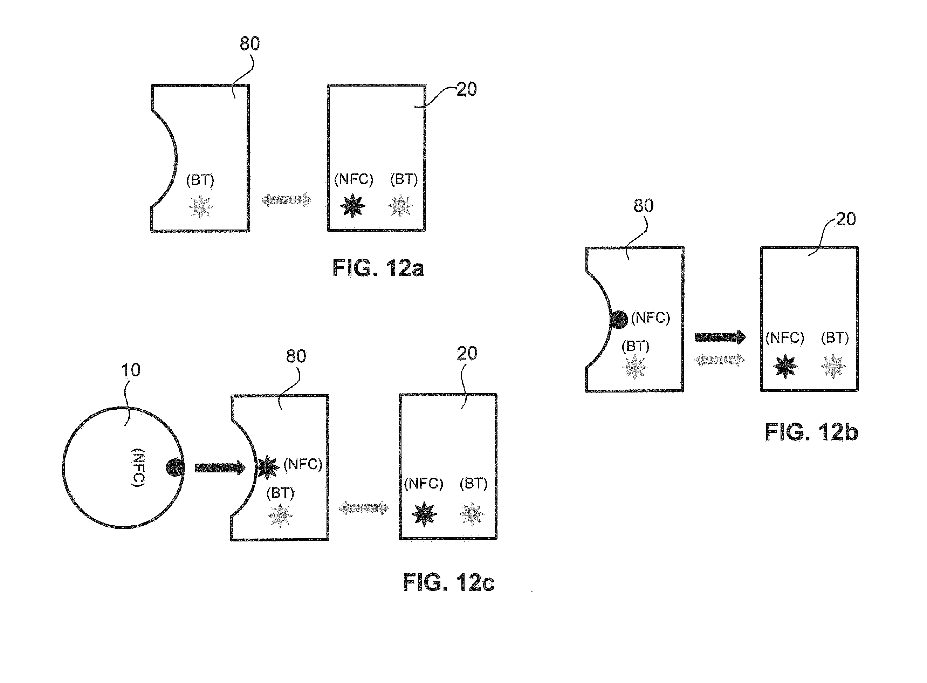

In den Ausführungsformen gemäss den Figuren 12a bis 12c erfolgt ein Datenaustausch zwischen Pumpe 80 und Smartphone 20. In Figur 12a ist die Pumpe 80 mit einem Bluetooth Modul ausgerüstet und kommuniziert so mit dem Smartphone 20. In Figur 12b weist die Pumpe 80 ein NFC Tag und ein Bluetooth Modul auf. Das NFT Tag konfiguriert ein Smartphone App, welches seinerseits mit der Pumpe 80 kommuniziert. In Figur 12c ist die Pumpe 80 mit einem aktiven NFC Leser/Schreiber und einem Bluetooth Modul ausgestattet. Die Bluetooth Verbindung wird mittels NFC konfiguriert, das Smartphone 20 kommuniziert anschliessend mit der Pumpe 80.

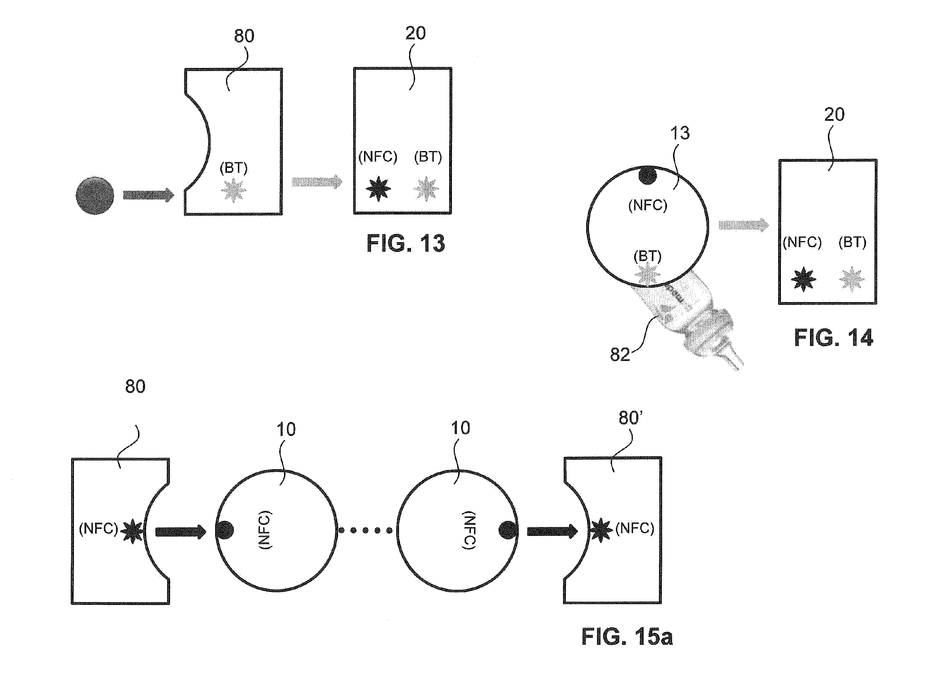

In Figur 13 wird eine produzierte Milchmenge erfasst. Die Pumpe 80 oder der Milchauffangbehälter oder ein zugehöriger Datenträger misst die Menge der produzierten Milch bzw. verfügt über andere Wege über diese Daten, beispielsweise durch manuelle Eingabe. Diese Daten werden an das Smartphone 20 übermittelt. Hier erfolgt die Übermittlung über die Pumpe 80. Wie oben dargelegt, kann sie auch über andere Produkte oder den Datenträger übermittelt werden.

In Figur 14 wird die abgegebene Milchmenge erfasst, beispielsweise wie in Figur 8 beschrieben, und an das Smartphone 20 übermittelt.

Die gemäss den Figuren 13 und 14 übermittelten Daten können in einem gemeinsamen Individuum-spezifischen Diagramm oder einer gemeinsamen Individuum-spezifischen Liste in der Basiseinheit, jedoch alternativ oder zusätzlich auch auf der Plattform 3, eingetragen und verwaltet werden. Werden Daten desselben Individuums über verschiedene Basiseinheiten 2 übermittelt, so ermöglicht die Plattform 3 die Zusammenführung dieser Daten in ein gemeinsames Diagramm oder in eine gemeinsame Liste. Diese Liste bzw. dieses Diagramm ermöglichen vielfältige Rückschlüsse und Hinweise zum weiteren Vorgehen. Es ist auch möglich, auf diese Weise Daten von mehreren Individuen zusammenzuführen und daraus Hinweise für einzelne Individuen oder andere Kreise zu geben.

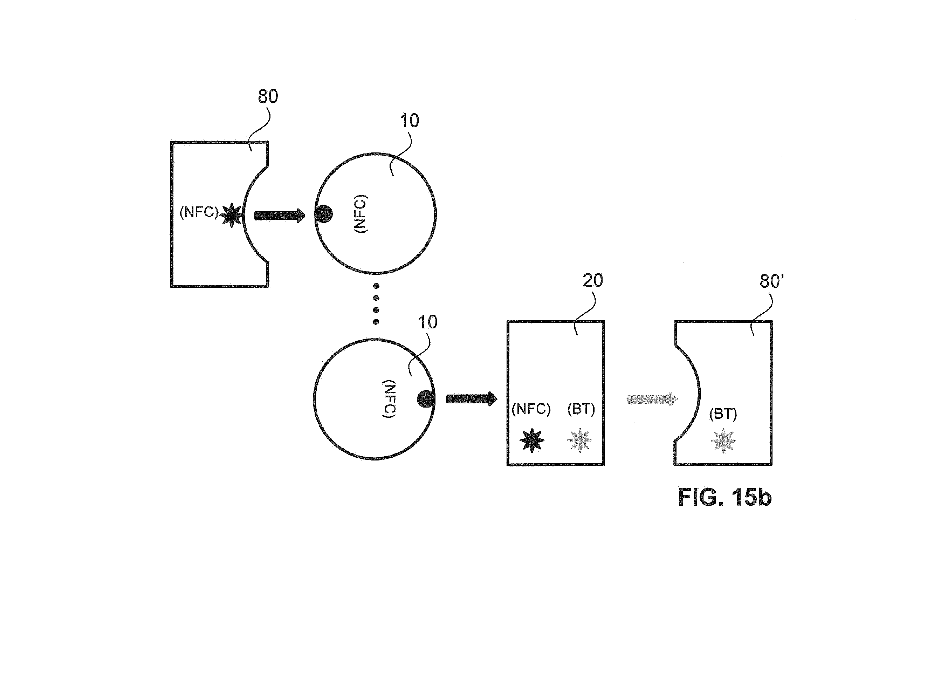

In den Figuren 15a und 15b erfolgt ein Datentransfer vom Spital nach Hause. D.h. ein Individuum, welches bereits im Spital Daten gesammelt hat und z.B. eine optimale Brustpumpeneinstellung gefunden hat, kann diese Daten mit nach Hause nehmen, um die dortigen Produkte ebenfalls optimal einzusetzen. In Figur 15a werden deshalb von der Spitalpumpe 80 oder der Basiseinheit im Spital Einstellungen der Spitalpumpe 80 und/oder andere Daten und Informationen auf den Chip 10 der Mutter gespeichert. Zuhause können diese Daten dann an die individuelle Pumpe 80' oder auf andere Produkte der Mutter übertragen werden. Alternativ oder zusätzlich können dieselben Daten zuhause auch mittels des Chips 1 in die individuelle Basiseinheit 20 der Mutter übertragen werden. Der Datentransfer zum Produkt, insbesondere zur Pumpe 80' kann dann auch über diese Basiseinheit 2 erfolgen. Dies ist in Figur 15b dargestellt.

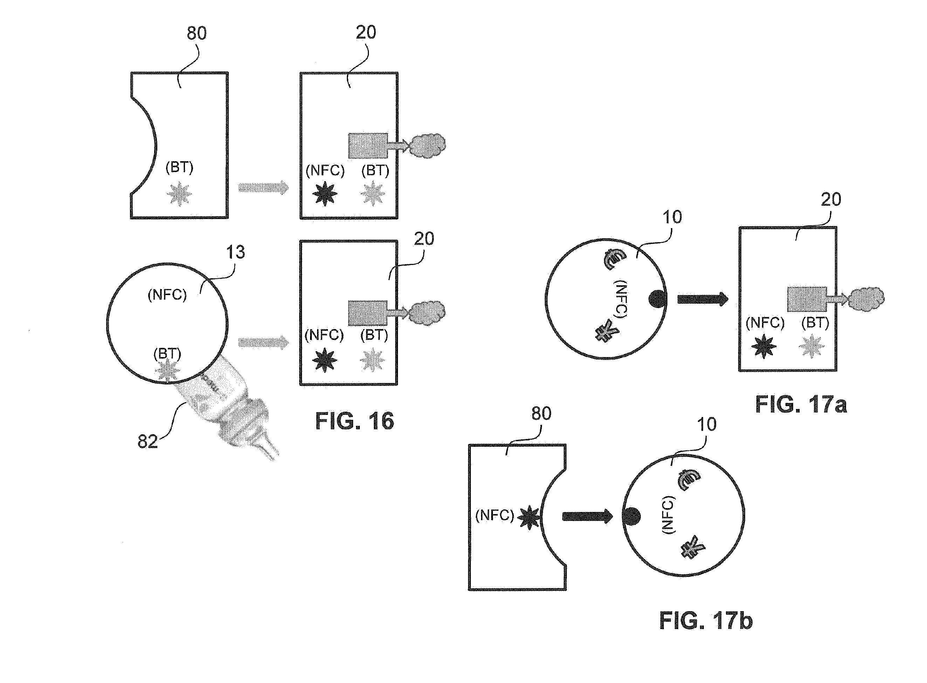

In Figur 16 ermöglicht das System einen optimierten Nachbezug von Ersatzmaterial. Das Produkt, hier die Pumpe 80, und/oder der Chip 10 wissen, welche Ersatzteile für das entsprechende Produkt erhältlich sind. Mittels Smartphone 20 und darauf installiertem App können die gewünschten Ersatzteile vom e-Shop 7 bezogen werden. Fehlbestellungen werden so vermieden.

In den Figuren 17a und 17b ist ein Bonusprogramm vorgeschlagen. Der Chip 10 gemäss Figur 17a ist mit einem Guthaben aufgeladen, welches mittels Basiseinheit, hier wiederum das Smartphone 20, im e-Shop eingelöst werden kann. Dies ermöglicht z.B. Geschenkgutscheine. In Figur 17b wird durch Gebrauch des Produkts, hier der Brustpumpe 80, ein Guthaben auf dem Chip 10 erarbeitet. Auch dieses kann anschliessend wieder eingelöst werden.

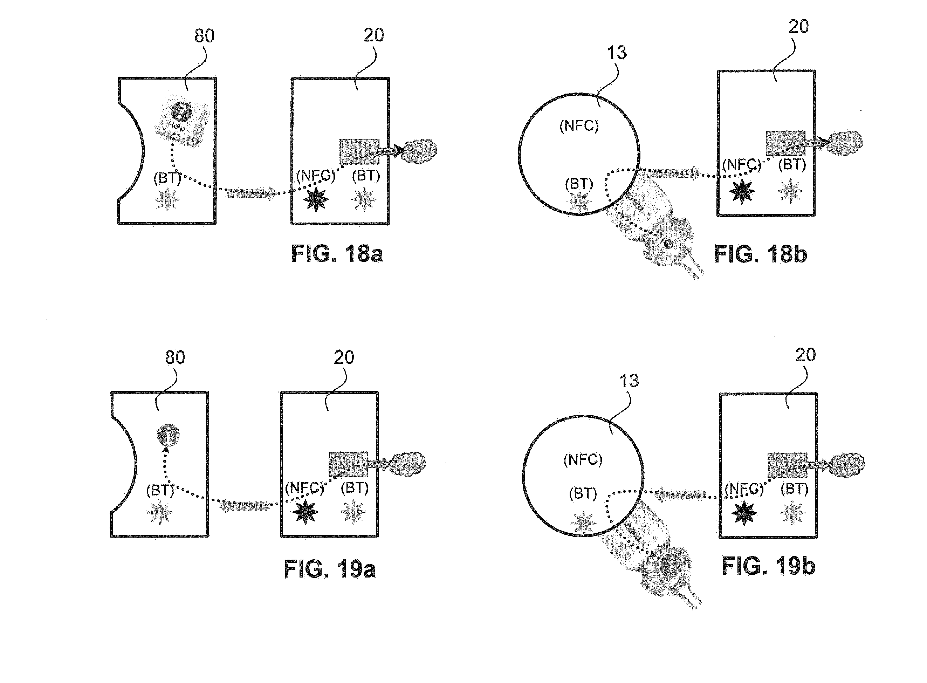

In den Figuren 18a und 18b lassen sich Signale vom Produkt 80, 82 an die Basiseinheit, hier das Smartphone 20, und von dort auf die Plattform 3 übermitteln. Dies ist insbesondere dann vorteilhaft, wenn Probleme beim Nutzer auftauchen. Eine Beratungsstelle oder ein Kundendienst kann so Signale direkt vom Produkt erhalten und überprüfen, wo der Fehler liegen könnte. Hierzu können der Hersteller oder andere Kreise auf der Plattform 3 entsprechende Informationstools oder automatische Fehlererkennungstools zur Verfügung stellen. Gemäss den Figuren 19a und 19b kann anschliessend oder auch proaktiv durch die Beratungsstelle, einen Kundendienst oder andere Kreise eine Nutzerunterstützung gegeben werden, wobei diese Unterstützung direkt das Produkt, hier die Pumpe 80, oder den Chip 10, beeinflusst oder derartige übermittelte Informationen direkt auf dem Produkt angezeigt werden.

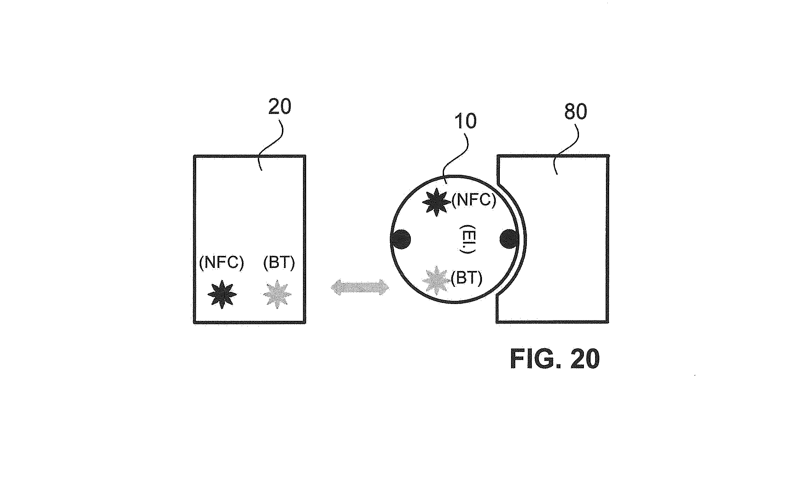

In Figur 20 ist eine Nachrüstung eines Produkts dargestellt, damit es überhaupt am erfindungsgemässen System teilhaben kann. Der Chip 10 ist mit einer entsprechenden Nachtrüst-Option versehen, welche durch Kontakt mit der Pumpe 80 oder einem anderen Produkt dessen Kommunikation mit dem Smartphone 20 und somit der Plattform 3 ermöglicht. Dieser Chip 10 kommuniziert mit dem Produkt vorzugsweise über ein elektrisches Interface und verbleibt somit vorzugsweise beim Produkt.

Das erfindungsgemässe System bietet in einem spezifischen Umfeld eine optimale Betreuung und Unterstützung von Nutzern.

**BEZUGSZEICHENLISTE**

| | | | |
|---|---|---|---|
| 10 | Datenträger | 5 | Unterstützungseinheit |
| 11 | erster Datenträgerring | 50 | Stillberatung |
| 12 | Datenträgerdeckel | 51 | Beratungsdienst |
| 13 | zweiter Datenträgerring | | |
| 100 | Anzeige | 6 | Hersteller |
| | | 60 | Kundendienst |
| 2 | Basiseinheit | 61 | Interne Bereiche |
| 20 | i-phone | | |
| 21 | Verbindungseinheit | 7 | e-Shop |
| 200 | Anzeige | | |
| | | 80 | Brustpumpe |
| 3 | Informations-und | 80' | zweite Brustpumpe |
| | Datenplattform | 81 | Brusthaube |
| 30 | Cloud | 82 | Milchsammelbehälter |
| | | 83 | Milchverabreichungsflasche |
| 4 | Produktanwender | | |
| 40 | Mutter | | |
| 41 | Helfer | | |

## Patentansprüche

1. Servicesystem im Umfeld der menschlichen Muttermilchgewinnung oder im Umfeld der medizinischen Drainage, wobei das System eine einem Individuum oder einer Entität zugeordnete Basiseinheit (2, 20) und mindestens einen mobilen Datenträger (10, 11, 12, 13) aufweist,
wobei der mobile Datenträger (10, 11, 12, 13) von einem Nutzer einerseits mit Produkten (80, 81, 82, 83) im genannten Umfeld und andererseits mit der Basiseinheit (2, 20) in Daten-kommunizierende Verbindung bringbar ist und
wobei die Basiseinheit (2, 20) vom Nutzer mit einer externen Informations- und Datenplattform (3) in kommunizierende Verbindung bringbar ist.

2. Servicesystem nach Anspruch 1, wobei das System die externe Informations- und Datenplattform (3) und mehrere Basiseinheiten (2, 20) umfasst und wobei diese Informations- und Datenplattform (3) Daten austauschbar, transformierbar und/oder optimierbar macht und den Basiseinheiten (2, 20) wieder zuordnungsbar macht, wobei die Daten mindestens einen der folgenden Bereiche betreffen: Saugpumpen, insbesondere Brustpumpen zum Abpumpen menschlicher Muttermilch oder Drainagepumpen; Muttermilch; Behandlung von Muttermilch; Babyfütterung; Muttermilchmanagement; Milchspeicherung.

3. Servicesystem nach einem der Ansprüche 1 oder 2, wobei die Mobilität des mobilen Datenträgers (10, 11, 12, 13) von der Basiseinheit und/oder von den Produkten (80, 81, 82, 83) unabhängig ist.

4. Servicesystem nach einem der Ansprüche 1 bis 3, wobei der mobile Datenträger (10, 11, 12, 13) vom Nutzer mit Produkten (80, 81, 82, 83) , insbesondere mit Saugpumpen, aus dem genannten Umfeld in daten-kommunizierende Verbindung bringbar ist.

5. Servicesystem nach einem der Ansprüche 1 bis 4, wobei die Produkte (80, 81, 82, 83) Saugpumpen, vorzugsweise Brustpumpen zum Abpumpen von menschlicher Muttermilch oder Thoraxdrainagepumpen oder Wunddrainagepumpen, umfassen.

6. Servicesystem nach einem der Ansprüche 1 bis 5, wobei die externe Informationsund Datenplattform (3) internetbasiert ist.

7. Servicesystem nach einem der Ansprüche 1 bis 6, wobei sich die externe Informations- und Datenplattform (3) mindestens teilweise in einer Cloud (30) befindet.

8. Servicesystem nach einem der Ansprüche 1 bis 7, wobei der mobile Datenträger (10, 11, 12, 13) einen bidirektionalen Datentransfer mit der Basiseinheit (2, 20) ermöglicht.

9. Servicesystem nach einem der Ansprüche 1 bis 8, wobei die Datenkommunikation zwischen mobilem Datenträger (10, 11, 12, 13) und Basiseinheit (2, 20) und/oder zwischen mobilem Datenträger (10, 11, 12, 13) und Saugpumpe (80) drahtlos erfolgt.

10. Servicesystem nach einem der Ansprüche 1 bis 9, wobei die Basiseinheit (2, 20) eine eigenständige Einheit mit Internetanschlussfunktion oder Teil eines Multifunktionskommunikationsmittels, beispielsweise eines Smartphones, ist.

11. Servicesystem nach einem der Ansprüche 1 bis 10, wobei die Basiseinheit (2, 20) einen bidirektionalen Informations- und Datentransfer mit mindestens einem Bereich der Informations- und Datenplattform (3) ermöglicht.

12. Servicesystem nach einem der Ansprüche 1 bis 11, wobei die Basiseinheit (2, 20) vom Nutzer mit den Produkten (80. 81, 82, 82) in Daten-kommunizierende Verbindung bringbar ist.

13. Servicesystem nach einem der Ansprüche 1 bis 12, wobei die Basiseinheit (2, 20) eine Auflagefläche zur Auflage des mobilen Datenträgers (10, 11, 12, 13) zum Zwecke des Datentransfers aufweist.

14. Basiseinheit (2, 20) eines Servicesystems gemäss einem der Ansprüche 1 bis 13.

15. Mobiler Datenträger (10, 11, 12, 13) eines Servicesystems gemäss einem der Ansprüche 1 bis 13, wobei er vorzugsweise die äussere Form einer Spielmarke (Chip), einer Münze oder einer Kreditkarte aufweist.

16. Mobiler Datenträger nach Anspruch 15, wobei er mindestens einen Sensor zur Detektion von Messwerten aufweist.

17. Mobiler Datenträger nach einem der Ansprüche 15 oder 16, wobei er zur Befestigung an einem der Produkte zwecks daten-kommunizierender oder messwertdetektierender Verbindung ausgebildet ist.

18. Produkt, insbesondere Saugpumpe, zur Verwendung in einem Servicesystem gemäss einem der Ansprüche 1 bis 13, wobei das Produkt zur Aufnahme des mobilen Datenträgers zwecks daten-kommunizierender oder messwertdetektierender Verbindung ausgebildet ist.
